**Europäisches Patentamt**

**European Patent Office**

**Office européen des brevets**

(19)

(11) Veröffentlichungsnummer: **0 182 824**
**B1**

# (12) EUROPÄISCHE PATENTSCHRIFT

(45) Veröffentlichungstag der Patentschrift:
**14.09.88**

(21) Anmeldenummer: **85902466.3**

(22) Anmeldetag: **22.05.85**

(86) Internationale Anmeldenummer:
**PCT/DE 85/00173**

(87) Internationale Veröffentlichungsnummer:
**WO 85/05279 (05.12.85 Gazette 85/26)**

(51) Int. Cl.⁴: **A 61 N 1/365**

(54) **BELASTUNGSABHÄNGIG FREQUENZVARIABLER HERZSCHRITTMACHER.**

(30) Priorität: **24.05.84 DE 3419439**

(43) Veröffentlichungstag der Anmeldung:
**04.06.86 Patentblatt 86/23**

(45) Bekanntmachung des Hinweises auf die Patenterteilung:
**14.09.88 Patentblatt 88/37**

(84) Benannte Vertragsstaaten:
**AT BE CH DE FR GB IT LI LU NL SE**

(56) Entgegenhaltungen:
**EP - A - 0 059 868**
**EP - A - 0 096 464**
**EP - A - 0 133 828**
**FR - A - 2 308 352**
**FR - A - 2 442 631**

(73) Patentinhaber: **ALT, Eckhard, Gartenstrasse 12A,**
**D-8000 München 40 (DE)**

(72) Erfinder: **ALT, Eckhard, Gartenstrasse 12A,**
**D-8000 München 40 (DE)**

(74) Vertreter: **Haft, Berngruber, Czybulka,**
**Postfach 14 02 46 Hans-Sachs-Strasse 5,**
**D-8000 München 5 (DE)**

ACTORUM AG

**Beschreibung**

Die Erfindung bezieht sich auf einen Herzschrittmacher gemäss dem Oberbegriff des Patentanspruches 1.

Neben der Registrierung der nur bei einem Bruchteil von Herzschrittmacherträgern vorhandenen normalen P-Wellen, das sind die elektrischen Signale des Herzvorhofes und deren Verwendung als Steuergrösse für Herzschrittmacher wurde in der Vergangenheit auf vielseitige Weise versucht, über die Wahrnehmung eines bestimmten biologischen Signales eine Anpassung der Stimulationsfrequenz eines Herzschrittmachers unter körpericher Belastung zu erreichen. Als biologische Signale, die für eine Frequenzvariation des Herzschrittmachers geeignet sind, wurden insbesondere der Säure- oder pH-Wert des venösen Blutes, die zentralvenöse Sauerstoffsättigung, die Atemfrequenz, das QT-Intervall, das ist die Dauer der elektrischen Erregung und Erregungsrückbildung der Herzkammer, und die zentralvenöse Bluttemperatur vorgeschlagen, Herzschrittmacher mit Verwendung der Atemfrequenz und des QT-Intervalls zur Frequenzveränderung befinden sich derzeit in Entwicklung oder klinischer Erprobung. Bei diesen beiden frequenzvariablen Herzschrittmachern können sich jedoch unliebsame unkontrollierte Tachykardien einstellen, die unter Umständen den Herzschrittmacherträger akut gefährden können. Darüberhinaus sind diese beiden Systeme als Einkammersysteme bisher nur für den Einsatz im Ventrikel geeignet und werden teilweise erheblich durch gängige Medikamente gestört, die auf den Elektrolyt- und Membranstoffwechsel einwirken, so z.B. Betablocker, Diuretika, Antiarrhythmika und Digitalis.

Zur Variation der Stimulationsfrequenz des Herzschrittmachers bietet sich als einfaches biologisches Signal, das als Steuer- und Regelparameter verwendet werden kann, die zentralvenöse Bluttemperatur an, wie dieses bereits in der DE-OS 26 09 365 vorgeschlagen worden ist. Ein temperaturgesteuerter Herzschrittmacher der dort beschriebenen Art wird zwar noch nicht in der Praxis verwendet, hätte jedoch den Vorteil, dass der hierzu benötigte Temperaturfühler sehr einfach und kleinbauend ist und in der Nähe der Stimulationselektrode direkt in die selbst als Elektrode bezeichnete Herzsonde eingebaut werden kann. In der genannten Offenlegungsschrift ist vorgeschlagen, die Stimulationsfrequenz gleichsinnig mit der Bluttemperatur einzustellen, d.h. bei einer Erhöhung der Bluttemperatur eine entsprechend höhere Stimulationsfrequenz vorzusehen, wobei eine lineare Abhängigkeit dieser beiden Grössen in Teilbereichen, z.B. zwischen 37°C und 39°C nicht ausgeschlossen wird.

Ein ähnlicher frequenzvariabler Herzschrittmacher, der ebenfalls in Abhängigkeit der zentralvenösen Bluttemperatur arbeitet, ist auch in der US-PS 4 436 092 vorgeschlagen worden. Die Bluttemperatur wird mit Hilfe eines Temperaturfühlers erfasst, der an einer transvenösen Zuleitung für eine Stimulationselektrode in der Nähe dieser Elektrode angeordnet ist. Temperaturfühler und Stimulationselektrode sind in den Ventrikel des Herzens eingeführt. Aufgrund von Versuchen wird eine mathematische Beziehung zwischen Bluttemperatur und Herzfrequenz bei einem normal funktionierenden Herzen unter Belastung bestimmt und in einer Logikschaltung als Kennlinie gespeichert. Die Stimulationsfrequenz wird nach Massgrösse dieser Kennlinie in Abhängigkeit der durch einen Temperaturfühler erfassten Bluttemperatur gesteuert. Die Steuerung nach der gespeicherten Kennlinie erscheint zwar mathematisch genauer als das allgemeine Steuerprinzip gemäss der DE-OS 26 09 365, ermöglicht jedoch ebenfalls keine Stimulation, die auf den physiologischen Zustand des Herzschrittmacherträgers optimal angepasst werden kann.

In Untersuchungen, die der Anmelder an einer Vielzahl von gesunden Menschen ohne Herzschrittmacher durchgeführt hat, konnte festgestellt werden, dass der Verlauf der Bluttemperatur und der Herzfrequenz unter Belastung sich weitgehend parallel verhalten, und zwar unabhängig von der jeweiligen Leistungsfähigkeit der Testperson. Eine Steuerung eines Herzschrittmachers in Abhängigkeit der Bluttemperatur entspricht daher tatsächlich den Bedingungen, die an einen Herzschrittmacher gestellt werden müssen, und zwar:

1. eine definierte Korrelation von Bluttemperatur und Herzfrequenz, die als praktisch linear angenommen werden kann und

2. eine intraindividuelle Reproduzierbarkeit dieser Korrelation, da das Verhältnis von Bluttemperatur zu Herzfrequenz weitgehend unabhängig von dem individuellen Leistungsvermögen ist.

Ausserdem kann die Bluttemperatur mit z.B. Halbleiter- oder Chip-Thermistoren leicht, konstant und mit grosser Langzeitgenauigkeit gemessen werden, wobei die Ansprechempfindlichkeit derartiger Temperaturfühler sehr hoch ist. Ausserdem haben diese Temperaturfühler nur einen geringen Energiebedarf und können, wie bereits oben erwähnt, wegen ihrer kleinen Baugrösse direkt in die Elektrode eingebaut werden.

Die Variation der Herzfrequenz unter Belastung in Abhängigkeit der erwähnten Korrelation kann jedoch in bestimmten Fällen wiederum zu unliebsamen und den Herzschrittmacherträger akut gefährenden Tachykardien führen. So kommt es z.B. bei Fieberzuständen und bei der Spontanvariation der Körpertemperatur im tagesrhythmischen Zyklus zu Änderungen der Bluttemperatur, die bei einem gesunden Menschen ohne Herzschrittmacher zwar auch von einer Änderung der Herzfrequenz begleitet werden, wobei diese Änderung dann allerdings nicht mehr der oben erwähnten Korrelation unter körperlicher Belastung folgt.

Der Erfindung liegt die Aufgabe zugrunde, einen Herzschrittmacher der eingangs genannten Art zu schaffen, der imstande ist, zwischen physiologisch bedingten Bluttemperaturänderungen im Ruhestand einerseits und auf körperliche Belastungen zurückzuführenden Bluttemperaturänderungen andererseits zu unterscheiden und die Stimulationsfolgefrequenz entsprechend unterschiedlich an die Bluttemperaturänderungen anzupassen.

Diese Aufgabe ist gemäss der Erfindung durch die im kennzeichnenden Teil des ersten Patentanspruches angegebenen Merkmale gelöst.

Die Erfindung basiert demnach auf der Erkenntnis, dass Belastungen auf den Herzschrittmacherträger je nach dessen physiologischem Zustand unterschiedlich bewertet werden müssen. So ist z.B. die Steigerung der Bluttemperatur pro Zeitintervall bei normaler Belastung, wie z.B. beim Treppensteigen unterschiedlich zu derjenigen, die z.B. zu Beginn eines Fiebers beim Herzschrittmacherträger auftritt. In einer Logik des Herzschrittmachers wird infolgedessen diese Steigerung der Bluttemperatur pro Zeiteinheit überwacht und ausgewertet sowie dann als Auswahlkriterium für den Wert der Stimulationsfrequenz herangezogen. Für die Abhängigkeit der Herzfrequenz von der Bluttemperatur kann z.B. in einem Matrixspeicher ein Kennlinienfeld vorgegeben sein, deren einzelne Kennlinien mehreren physiologischen Zuständen des Patienten entsprechen. Entsprechend den Ergebnissen der Auswerteschaltung wird die Stimulationsfrequenz entsprechend einer der Kennlinien innerhalb des Kennlinienfeldes gesteuert.

Es hat sich herausgestellt, dass es zur Steuerung der Stimulationsfrequenz ausreichend ist, eine absolute Basiskennlinie und ein relatives Belastungskennlinienfeld zu verwenden. Die Basiskennlinie ordnet eine Absoluttemperatur einer festen Frequenz zu, bei der körperliche Belastungen des Herzschrittmacherträgers nicht ausschlaggebend für eine Bluttemperatursteigerung sind. Dies tritt z.B. im Fieberzustand oder bei den erwähnten tagesrhythmischen Schwankungen wie der Temperatur- und Herzfrequenzabsenkung während des Schlafes auf. Die Belastungskennlinien sind relative Kennlinien, die ausgehend von einem Arbeitspunkt auf der Basiskennlinie einen Herzfrequenz/Bluttemperaturverlauf unter körperlicher Belastung aufweisen, die einem relativen Bluttemperaturanstieg eine Herzfrequenzerhöhung zuordnen und auf die der Herzschrittmacher bei signifikantem Anstieg der Bluttemperatur pro Zeitintervall umschaltet, z.B. bei einem Anstieg von mindestens 0,04 Grad pro Minute. Diese Kennlinie berücksichtigt demnach relative Bluttemperaturänderungen.

Liegt der Arbeitspunkt des Herzschrittmachers auf der Basiskennlinie, so ist der gemessenen absoluten Bluttemperatur eine feste Herzfrequenz zugeordnet, z.B. eine Frequenz von 70 Schlägen pro Minute bei einer Bluttemperatur von 37°C oder eine Herzfrequenz von 95 Schlägen pro Minute bei einer Bluttemperatur von 38,5°C entsprechend einem Fieberzustand. In beiden Fällen befindet sich der Herzschrittmacherträger in Ruhe. Tritt jetzt eine körperliche Belastung auf, dann steigt die Bluttemperatur signifikant stärker pro Zeitintervall an als normalerweise im Ruhezustand. Der Herzschrittmacher schaltet in diesem Falle auf die von dem Arbeitspunkt ausgehende Belastungskennlinie, so dass die Stimulationsfrequenz entsprechend dieser Kennlinie gesteuert wird. Nach Ende der körperlichen Belastung fällt die Bluttemperatur und die Stimulationsfrequenz wird auf dieser Belastungskennlinie in Richtung auf die Basiskennlinie abgesenkt. Dies erfolgt solange, bis der Bluttemperaturabfall pro Zeitintervall kleiner als ein unterer Grenzwert ist, der so gewählt ist, dass sich die Bluttemperatur praktisch nicht mehr ändert. Dies wird in der Regel bei einer Bluttemperatur der Fall sein, die wieder der Ruhetemperatur oder einem sehr nahe um diese Ruhetemperatur liegenden Wert entspricht. Der Herzschrittmacher schaltet dann wieder auf die Basiskennlinie um, wobei selbstverständlich diese Umschaltung physiologisch zuträglich erfolgt, d.h. keine Sprünge für die Herzfrequenz aufweist. Als Kriterium für die Umschaltung von der jeweiligen Belastungskennlinie zurück in Richtung auf die Basiskennlinie kann neben dem erwähnten Bluttemperaturabfall auch noch eine physiologisch angepasste Zeitspanne dienen, die zwischen einigen Minuten und einer Stunde, vorzugsweise bei 30 Minuten liegt, wenn sich die gemessenen Temperaturwerte bzw. deren zeitlichen Änderungen nicht wesentlich ändern. Beide Kriterien verhindern zuverlässig eine für den Herzschrittmacherträger unverträgliche Tachykardie. Wird der Herzschrittmacherträger z.B. über längere Zeit körperlich belastet, so wird der Herzschrittmacher wie oben beschrieben entsprechend einer Belastungskennlinie gesteuert. Im Laufe der an- und absteigenden körperlichen Belastung kann es über längere Zeit zu einem Gleichgewichtszustand kommen, bei der der Arbeitspunkt des Herzschrittmachers auf einer Belastungskennlinie über längere Zeit innerhalb enger Grenzen verbleibt. Ein solcher Gleichgewichtszustand stellt sich z.B. bei einer längeren Wanderung oder einer leichten Bergwanderung ein. Dieser Gleichgewichtszustand wird von der Logik des Herzschrittmachers – gegebenenfalls nach der erwähnten physiologisch zuträglichen Zeitspanne von 30 Minuten – als Umschaltkriterium in Richtung auf die Basiskennlinie gewertet. Innerhalb der Herzschrittmacherlogik wird demzufolge ein Übergangsprogramm für diesen Übergang eingeleitet, so dass z.B. in physiologisch zuträglichem Masse die Herzfrequenz abgesenkt wird. Hierdurch wird auch die Pumpleistung des Herzens verringert. Wenn der Herzschrittmacherträger jedoch weiterhin unter derselben körperlichen Belastung steht, aufgrund der abfallenden Herzfrequenz jedoch nur noch eine geringere Herzpumpleistung aufbringt, reagiert der Körper mit einer relativen Temperatursteigerung, aufgrund der die Logik des Herzschrittmachers diesen automatisch wieder auf eine Belastungskennlinie umschaltet. Diese Umschaltung erfolgt innerhalb kurzer Zeit ohne merkliche Unzuträglichkeit für den Herzschrittmacherträger z.B. in Form von Herzklopfen.

Falls der Herzschrittmacherträger keiner weiteren körperlichen Belastung mehr ausgesetzt ist, dann schaltet die Logik den Herzschrittmacher entsprechend einer Übergangsfunktion von der Belastungskennlinie auf die Basiskennlinie.

Der Verlauf der Basiskennlinie und der Belastungskennlinien ist im Prinzip frei wählbar, solange dieser Verlauf den physiologischen Zuständen des Herzschrittmacherträgers angepasst ist. Es hat sich herausgestellt, dass diese Kennlinien ohne weiteres als Geraden gewählt werden können, wobei dann die Steigung der Belastungskennlinien zwischen 40 und 120 Schlägen pro Minute und Grad gewählt wird. Für die Mehrzahl der Herzschrittmacherträger können diese Steigungen zu 80 bzw. 15 Schlägen pro Minute und Grad fest eingestellt werden. Der Verlauf der einzelnen Kennlinien kann in ihren höheren Bluttemperaturen zugeordneten Bereichen eine abnehmende Steigung aufweisen, was den physiologischen Zuständen besser entspricht. Prinzipiell können alle Belastungskennlinien parallel zueinander verlaufen, wodurch sich die Schaltung der internen Auswertung und Logik des Herzschrittmachers vereinfacht. In einem solchen Fall arbeitet man lediglich mit einer absoluten Basiskennlinie und einer relativen Belastungskennlinie, die je nach dem Arbeitspunkt des Herzschrittmachers parallel zur Abszisse, das ist die Bluttemperaturachse des Kennliniendiagramms, verschoben wird.

Die Logik, die die Funktion des Herzschrittmachers steuert, kann hinsichtlich mehrerer Parameter programmiert werden. So kann z.B. die Herzfrequenz in den Bereichen zwischen 50 und 180 Schlägen pro Minute limitiert werden; ebenso können als Minimal- und Maximalwert der Bluttemperatur z.B. Werte von 36 und 40°C eingegeben werden. Diese einstellbaren Grenzen dienen zur Anpassung des Betriebsbereiches des Herzschrittmachers individuell an den jeweiligen Träger.

Die Bluttemperatur wird bevorzugt getaktet gemessen, wobei die Messfrequenz vorzugsweise gleichzeitig mit dem Anstieg der Bluttemperatur pro Zeitintervall veränderbar ist. Auf diese Weise wird eine Änderung der Bluttemperatur bei körperlicher Belastung ohne Verzögerung durch zu lange Messintervalle schnell erkannt und dem physiologischen Zustand des Herzschrittmacherträgers angepasst werden.

In Versuchen hat sich gezeigt, dass intermittierende, plötzliche Änderungen der Bluttemperatur auftreten. Diese sind dadurch bedingt, dass der Temperaturfühler zeitweiligen Kontakt mit anderen Substanzen im Blut aufweist, z.B. direkt mit dem Herzmuskel, der die Änderungen der Bluttemperatur nicht in gleich schneller Weise wie Blut nachvollzieht. Dieser intermittierende Kontakt kann z.B. im Rahmen der mechanischen Lageänderung des Temperaturfühlers durch Atmung oder Herzkontraktion bedingt werden. Durch eine Glättung der Bluttemperaturmesswerte über eine Mittelwert-, Maximalwert- oder Minimalwertbildung können diese intermittierenden Schwankungen relativiert werden.

Um die Änderung der Bluttemperatur unter körperlicher Belastung mit Muskelarbeit mit Armen und Beinen in gleicher Weise zu erfassen, ist es notwendig, dass sich der Temperaturfühler im Bereich der Vorhofkammergrenze des Herzens befindet, wo eine gute Vermischung des venösen Blutes stattfindet. Durch die Plazierung des Temperaturfühlers 4 bis 8 cm hinter der Elektrodenspitze ist eine solche Elektrode gleichermassen zur Implantation in der Kammer als auch im Herzohr geeignet: Durch die Schlaufenbildung der Elektrodensonde bei Implantation im Herzohr gelangt der Temperaturfühler ebenfalls in die Vorhofkammergrenze.

Weitere Ausgestaltungen der Erfindung gehen aus den Unteransprüchen hervor. Die Erfindung ist in einem Ausführungsbeispiel anhand der Zeichnung näher erläutert. In der Zeichnung stellen dar:

Figur 1 ein Blockdiagramm eines Herzschrittmachers gemäss der Erfindung;

Figur 2 eine Schemadarstellung einer Elektrode mit unipolarer Stimulationselektrode und integriertem Temperaturfühler, wobei die Elektrode mit einem implantierten Schrittmachergehäuse zur Energieversorgung und Auswertung verbindbar ist;

Figur 3 ein Kennliniendiagramm für einen Herzschrittmacher gemäss der Erfindung mit einem eingezeichneten Betriebszyklus zur Darstellung der Arbeitsweise des Herzschrittmachers;

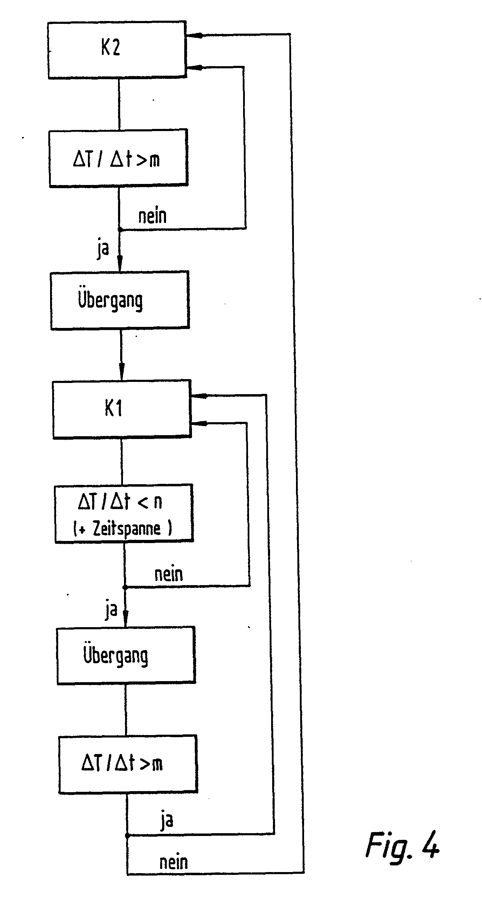

Figur 4 ein Flussdiagramm zur Erläuterung der Arbeitsweise eines Herzschrittmachers gemäss der Erfindung.

Ein Herzschrittmacher 1 weist eine Elektrode 2 auf, die an ihrer Spitze eine Stimulationselektrode 3 aufweist. In einem Abstand von ca. 4 bis 8 cm hinter der vorderen Spitze der Elektrode ist ein Thermistor als Temperaturfühler 4 vorgesehen. Die Elektrode wird in den Vorhof oder die Herzkammer eines Herzens 5 implantiert. Die Herzsonde ist mit einem implantierten Schrittmachergehäuse 6 verbunden, in dem eine Batterie 7, ein Impulsgenerator 8 zur Erzeugung der Stimulationsimpulse für die Elektrode 3, eine Auswerteschaltung 9, ein Speicher 10 und eine Logik 11 enthalten sind.

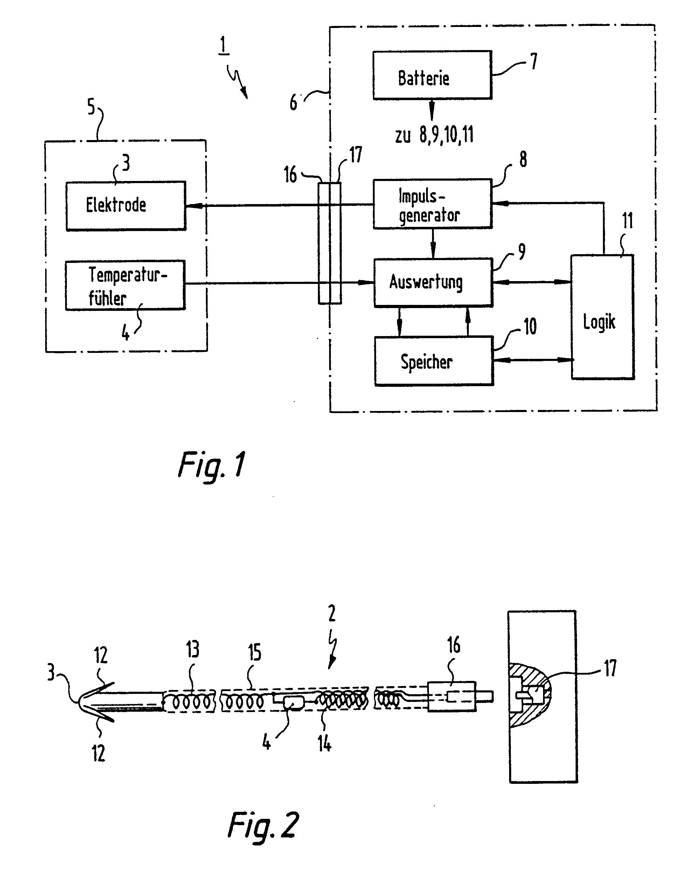

Die Elektrode 2 ist entsprechend Figur 2 als unipolare Elektrode ausgebildet, wobei die Stimulationselektrode 3 an ihrer Spitze mehrere Spreizelemente 12 aufweist, die zur Stabilisation der Elektrode dienen, so dass diese auch im Herzohr verankert werden kann. Der Anschluss der Stimulationselektrode erfolgt über eine eingängige leitende Wendel 13, die auch mit einem Anschluss des Thermistors 4 verbunden sein kann. Der andere Anschluss des Thermistors ist mit einer zweiten Wendel 14 verbunden. Die beiden Wendeln 13 und 14 sind gegeneinander elektrisch isoliert; die Elektrode 2 selbst ist mit einer Isolation 15 umgeben. Die Elektrode ist klein und flexibel genug, um in üblicher Weise implantiert werden zu können. Die beiden Wendeln 13 und 14 sind auf seiten des Gehäuses 6 mit einem mehrpoligen Stecker 16, in diesem Fale einem Koaxialstecker verbunden, der in einem korrespondierenden Adapter 17 auf seiten des Gehäuses 6 eingeführt werden kann. Der Koaxialstecker 16 ist hierbei so ausgebildet, dass die Elektrode auch mit solchen herkömmlichen Herzschrittmachern verbunden werden kann, die keine Möglichkeit zur Messung und Verarbeitung der Temperatur aufweisen. Im Koaxialstecker 16 ist noch eine Referenzschaltung in Halbbrückenausführung vorgesehen, die zur Eichung des Thermistors 4 auf Absoluttemperatur dient und ebenfalls über einen Pol des Mehrfachsteckers 16 mit Adapter 17 und Auswerteschaltung 9 verbunden ist. Auf diese Weise werden verschiedene Elektroden und Schrittmacher untereinander frei austauschbar. Anstelle einer unipolaren Stimulationselektrode kann selbstverständlich auch eine bipolare Stimulationselektrode verwendet werden, bei der Kathode und Anode in der Nähe der Spitze der Elektrode 2 angeordnet sind. Auch der elektrische Anschluss des Temperaturfühlers 4 ist beispielhaft und könnte z.B. auch über zwei gegeneinander isolierte separate Anschlussdrähte erfolgen.

Der Temperaturfühler 4 weist eine ausreichende Langzeitstabilität bei gleichzeitig hoher Auflösung von etwa 1/100stel Grad auf. Der Strombedarf für den Temperaturfühler ist gegenüber dem Strombedarf für die sonstigen Elemente des Schrittmachersystems sehr gering und hat somit praktisch keinen ins Gewicht fallenden Einfluss auf die Lebensdauer insbesondere der Batterie.

Der Temperaturfühler misst – gesteuert von der Logik 11 – die Bluttemperatur im Abstand von etwa ein bis zehn, bevorzugt fünf Sekunden. Die Temperaturwerte werden der Auswerte-Schaltung 9 zugeführt und in einen Speicher 10 eingeschrieben. Aus diesen gespeicherten Werten und den jeweiligen Messwerten berechnet die Auswerteschaltung 9 den Anstieg der Bluttemperatur pro Zeiteinheit. Auswerteschaltung und Speicher sind über bidirektionale Datenleitungen mit der Logik 11 verbunden. Diese Logik 11 legt fest, wie der Impulsgenerator 8 gesteuert wird.

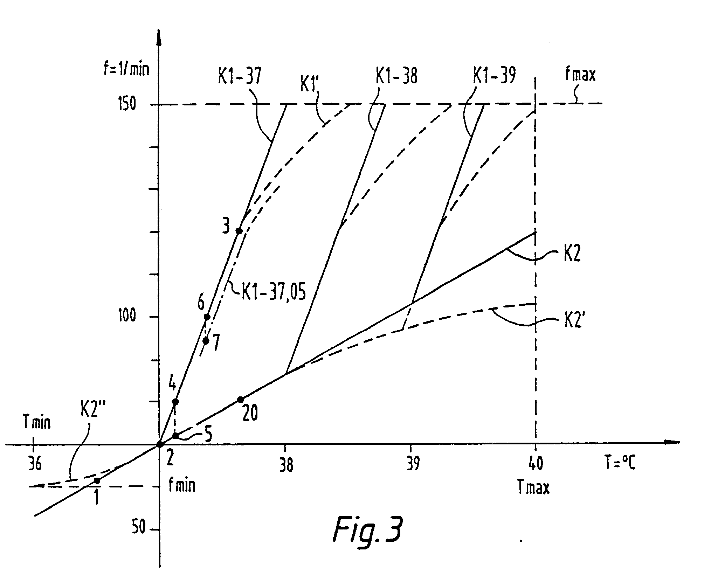

Die Steuerung des Impulsgenerators 8 durch die Logik 11 erfolgt anhand des in Figur 3 dargestellten Kennliniendiagrammes. Eine gerade Basiskennlinie K2 stellt eine absolute Beziehung zwischen Bluttemperatur und Herzfrequenz im Bereich von 36°C bis 40°C her, wobei die Herzschlagfrequenzen zwischen ca. 50 und 120 Schlägen pro Minute variieren. Dieser Basiskennlinie ist eine Kennlinienschar aus ebenfalls linearen Belastungskennlinien K1 überlagert, die jeweils von einem Arbeitspunkt auf der Basiskennlinie K2 ausgehen und in diesem Falle eine Steigung von 80 Schlägen pro Minute und Grad aufweisen. Sämtliche Belastungskennlinien sind zueinander parallel. In Figur 3 sind drei Belastungskennlinien K1–37, K1–38 und K1–39 eingezeichnet, die jeweils Bluttemperaturwerten von 37°C, 38°C bzw. 39°C zugeordnet sind. Basiskennlinie K2 und die Belastungskennlinien K1 können im Bereich höherer Temperaturen leicht gekrümmt sein, wie dieses durch K2', K1' angedeutet ist, so dass die Kennlinien in diesen Bereichen eine geringe Steigung aufweisen. Im Bereich zwischen 37°C und 36°C kann die Basiskennlinie K2 leicht abgeflacht sein, was durch K1" angedeutet ist. Minimal- und Maximalwerte für die Herzfrequenz und die Bluttemperatur sind frei programmierbar und in diesem Falle mit fmin bei 60 Schläge pro Minute, fmax bei 150 Schläge pro Minute, Tmin bei 36°C und Tmax bei 40°C eingetragen. Diese Werte bestimmen den Betriebsbereich des Herzschrittmachers.

In Figur 3 ist ein Betriebszyklus des Herzschrittmachers durch auf den Kennlinien aufgezeichnete Punkte 1 bis 7 dargestellt. Befindet sich der Herzschrittmacherträger in Ruhe, so wird die Herzfrequenz entsprechend der Kennlinie K2 gesteuert. Beim Aufwachen am Morgen ist die Bluttemperatur 36,5°C bei einer Herzfrequenz von 60 Schlägen pro Minute (Punkt 1). Die Bluttemperatur erhöht sich anschliessend entsprechend des üblichen tagesrhythmischen Wechsels auf 37°C bei einer Herzfrequenz von 70 Schlägen pro Minute (Punkt 2). Bleibt der Herzschrittmacherträger weiterhin in Ruhe, d.h. er unterwirft sich keinen merklichen körperlichen Belastungen, so steigt die Bluttemperatur pro Zeitintervall nur geringfügig, so dass die interne Logik 11 die Stimulationsfrequenz entsprechend der Basiskennlinie K2 steuert. Die Herzfrequenz bleibt während dieser Steuerung durchaus nicht konstant und kann auch höhere Werte als die Grundfrequenz bei 37°C annehmen, was durch den Punkt 20 angedeutet ist. Der Anstieg der Bluttemperatur pro Zeiteinheit ist hierbei jedoch nur sehr gering, solange der Herzschrittmacherträger nicht körperlich belastet wird. Wird der Herzschrittmacherträger jedoch körperlich belastet, steigt z.B. Treppen hinauf, so steigt die Bluttemperatur innerhalb eines bestimmten Zeitintervalls wesentlich schneller an. Erreicht der Anstieg der Bluttemperatur pro Zeiteinheit eine gewisse Grenze, z.B. 0,04 Grad pro Minute, dann schaltet der Herzschrittmacher auf eine Belastungskennlinie K1 um. Betrug die Bluttemperatur zu diesem Zeitpunkt 37°C, so erfolgt eine Umschaltung auf die Belastungskennlinie K1–37. Es sei angenommen, dass die Bluttemperatur auf etwas über 37,6°C ansteigt (Punkt 3), wodurch die Herzfrequenz auf 120 Schläge pro Minute angehoben wird. Nach Ende der körperlichen Belastung fällt entsprechend des individuellen Leistungsvermögens des Herzschrittmacherträgers die Bluttemperatur mehr oder minder schnell ab, wobei die Stimulationsfrequenz entsprechend dieser Kennlinie K1–37 heruntergesteuert wird, bis etwa der Punkt 4 bei einer Bluttemperatur von etwas über 37,1°C bei 80 Herzschlägen pro Minute erreicht wird. An diesem Punkt 4 ändert sich die Bluttemperatur nurmehr geringfügig mit der Zeit und bleibt längere Zeit entsprechend der Erholungsfähigkeit des Herzschrittmacherträgers annähernd konstant. Dieser Arbeitspunkt 4 auf der Kennlinie K1 liegt bei ansonsten gesunden Menschen regelmässig in der Nähe der Normaltemperatur von in diesem Falle 37°C. Die Logik 11 schaltet dann den Herzschrittmacher langsam auf die Basiskennlinie K2 um, so dass der Arbeitspunkt 5 auf dieser Kennlinie mit einer Frequenz von ca. 72 Schlägen pro Minute erreicht wird. Anschliessend wird die Stimulationsfrequenz weiter nach der Basiskennlinie K2 eingestellt, solange der Herzschrittmacherträger keinen körperlichen Belastungen ausgesetzt ist.

Ausserhalb dieses normalen Betriebszyklus kann es jedoch eintreten, dass sich die Bluttemperatur trotz körperlicher Belastung nicht wesentlich ändert, wenn sich nämlich ein physiologischer Gleichgewichtszustand unter körperlicher Belastung einstellt. Es sei angenommen, dass dies im Arbeitspunkt 6 auf der Kennlinie K1–37 erfolgt, d.h. dass sich die Bluttemperatur nach einer anfänglichen Erhöhung auf etwa 37,6°C im Arbeitspunkt 3 auf 37,4°C bei einer Herzfrequenz von 100 Schlägen pro Minute absenkt und sich dort stabilisiert. Dieser Zustand ist für die Logik an sich ein Umschaltkriterium auf die Basiskennlinie K2. Nach einer internen festgelegten physiologisch verträglichen Zeitspanne von z.B. 30 Minuten wird daher über die Logik die Herzfrequenz langsam abgesenkt, bis ein Arbeitspunkt 7 bei etwa 95 Schlägen pro Minute und der gleichen Temperatur erreicht wird. Ist der Herzschrittmacherträger tatsächlich noch der gleichen körperlichen Belastung wie zuvor ausgesetzt und verringert die Herzfrequenzerniedrigung die Pumpleistung des Herzens, so wird der Körper dieses ausgleichen und reagiert mit einer entsprechenden Temperaturerhöhung. Diese Temperaturerhöhung wird von dem Temperaturfühler praktisch unverzögert festgestellt und führt dazu, dass die Rückführung auf die Basiskennlinie K2 unterbrochen wird. Der Herzschrittmacher wird entweder direkt auf den Arbeitspunkt 6 zurückgeführt oder nach einer neuen Kennlinie entsprechend dem Temperaturwert in den Arbeitspunkt 7 gesteuert. In diesem Falle ist es die strichpunktiert eingezeichnete Kennlinie K1–37,05, die von einem Arbeitspunkt auf der Basiskennlinie K2 bei 37,05°C ausgeht. Eine solche Steuerung ist physiologisch sinnvoll, da sich entsprechend der Leistungsfähigkeit der Organismus des Herzschrittmacherträgers die Herzfrequenz im neuen optimalen Temperatur-Niveau wieder einregelt. Lässt die körperliche Belastung im weiteren Verlauf nach, so wird der Herzschrittmacher wie oben beschrieben, längs einer Belastungslinie auf die Basiskennlinie zurückgeführt.

Die Umschaltung von der Basiskennlinie auf eine Belastungskennlinie, zwischen den Belastungskennlinien und von einer Belastungskennlinie auf die Basiskennlinie geschieht selbstverständlich nicht abrupt, sondern gesteuert durch eine Übergangsfunktion entsprechend eines internen Regelprozesses im Verlauf von wählbaren Zeitintervallen. In Figur 5 ist die Betriebsweise des Herzschrittmachers in einem Ablaufdiagramm erläutert.

Die Messung, Speicherung und elektronische Auswertung der Bluttemperatur in dem beschriebenen Sinne kann zum einen dazu dienen, einen frequenzadaptiven Herzschrittmacher in Abhängigkeit der Bluttemperatur zu steuern. Die Bluttemperatur und die daraus ausgewerteten Resultate können darüberhinaus aufgrund ihrer direkten Korrelation zu hämodynamischen Parametern auch in günstiger Weise dazu geeignet sein, die Effektivität eines Herzschrittmachers zu kontrollieren, der als Steuerparameter nicht die zentralvenöse Bluttemperatur benutzt, sondern einen der oben erwähnten physiologischen Parameter wie zentralvenöse Sauerstoffsättigung, Atmungsfrequenz und dergleichen.

## Patentansprüche

1. Herzschrittmacher mit einer Steuervorrichtung zum automatischen Anpassen der Stimulationsfolgefrequenz an die Belastung des Patienten mit einem Temperaturfühler zum Erfassen der Temperatur des venösen Blutes im Herzen, der an einer transvenösen Zuleitung für eine Stimulationselektrode in der Nähe der Stimulationselektrode angeordnet ist dergestalt, dass er mit der Stimulationselektrode in den Vorhof oder Ventrikel des Herzens einführbar ist, und mit einer an den Temperaturfühler angeschlossenen Logikschaltung, die die Stimulationsfolgefrequenz nach Massgabe einer gespeicherten Kennlinie in Abhängigkeit von der durch den Temperaturfühler erfassten Bluttemperatur steuert, dadurch gekennzeichnet, dass die Logikschaltung (11) fortlaufend aus den durch den Temperaturfühler (4) erfassten Bluttemperaturen die aktuelle zeitliche Änderung der Bluttemperatur ermittelt und dass die Logikschaltung (11) die Stimulationsfolgefrequenz im Falle, dass die zeitliche Änderung der Bluttemperatur einen bestimmten Minimalwert nicht überschreitet, nach Massgabe einer Herzfrequenz-Bluttemperatur Basiskennlinie (K2), die dem körperlichen Ruhezustand entspricht, im Falle, dass die zeitliche Änderung der Bluttemperatur diesen Minimalwert überschreitet, hingegen nach Massgabe von Herzfrequenz-Bluttemperatur Belastungskennlinien (K1), die körperlichen Belastungszuständen entsprechen, steuert, wobei jede der Belastungskennlinien (K1) jeweils bei einem anderen Arbeitspunkt auf der Basiskennlinie (K2) beginnt und ansonsten bei ein und derselben Bluttemperatur jeweils eine höhere Stimulationsfolgefrequenz aufweist als die Basiskennlinie (K2) und alle Belastungskennlinien (K1) durchgehend eine grössere Steigung aufweisen als die Basiskennlinie (K2).

2. Herzschrittmacher nach Anspruch 1, dadurch gekennzeichnet, dass die Basiskennlinie (K2) und die Belastungskennlinien (K1) Geraden sind, wobei die Steigung der Belastungskennlinien (K1) erheblich grösser ist als diejenige der Basiskennlinie (K2).

3. Herzschrittmacher nach Anspruch 2, dadurch gekennzeichnet, dass die Steigung der Belastungskennlinien (K1) im Bereich zwischen 40 und 120 Stimulationen pro Minute und Grad und die Steigung der Basiskennlinie (K2) im Bereich zwischen 5 und 25 Stimulationen pro Minute und Grad liegen.

4. Herzschrittmacher nach Anspruch 3, dadurch gekennzeichnet, dass die Steigung der Belastungskennlinien (K1) 80 Stimulationen pro Minute und Grad und die Steigung der Basiskennlinie (K2) 15 Stimulationen pro Minute und Grad beträgt.

5. Herzschrittmacher nach einem der vorangehenden Ansprüche, dadurch gekennzeichnet, dass die Steigung der Belastungskennlinien (K1) und der Basiskennlinie (K2) im Bereich der höheren Bluttemperaturen abnimmt.

6. Herzschrittmacher nach einem der vorangehenden Ansprüche, dadurch gekennzeichnet, dass alle Belastungskennlinien (K1) parallel zueinander verlaufen.

7. Herzschrittmacher nach einem der vorangehenden Ansprüche, dadurch gekennzeichnet, dass die Logikschaltung (11) von der Basiskennlinie (K2) zu der von dem jeweiligen Arbeitspunkt der Basiskennlinie (K2) ausgehenden Belastungskennlinie (K1) wechselt oder auf der Belastungskennlinie (K1) bleibt, wenn die zeitliche Änderung der Bluttemperatur einen oberen Grenzwert überschreitet, und dass die Logikschaltung (11) von der Belastungskennlinie (K1) auf die Basiskennlinie (K2) wechselt, wenn die zeitliche Änderung der Bluttemperatur einen unteren Grenzwert für eine vorgegebene Zeitspanne unterschreitet.

8. Herzschrittmacher nach Anspruch 7, dadurch gekennzeichnet, dass der obere Grenzwert bei 0,04 Grad pro Minute liegt.

9. Herzschrittmacher nach einem der vorangehenden Ansprüche, dadurch gekennzeichnet, dass der Herzschrittmacher (1) eine Auswerteschaltung (9) enthält, die in vorgegebenen Zeitabschnitten die zeitliche Änderung der Bluttemperatur ermittelt, wobei die ermittelten Werte wie auch die Werte der Bluttemperatur in einem Speicher (10) gespeichert werden.

10. Herzschrittmacher nach einem der vorangehenden Ansprüche, dadurch gekennzeichnet, dass der Übergang zwischen den Kennlinien (K1, K2) ohne Sprünge in der Stimulationsfolgefrequenz erfolgt.

11. Herzschrittmacher nach einem der vorangehenden Ansprüche, dadurch gekennzeichnet, dass die vom Bluttemperaturfühler (4) erfasste Bluttemperatur periodisch mit einer vorgegebenen Folgefrequenz abgetastet wird.

12. Herzschrittmacher nach Anspruch 11, dadurch gekennzeichnet, dass die Folgefrequenz, mit der die erfasste Bluttemperatur abgetastet wird, gleichsinnig mit dem zeitlichen Anstieg der Bluttemperatur verändert wird.

13. Herzschrittmacher nach einem der vorangehenden Ansprüche, dadurch gekennzeichnet, dass der Temperaturfühler (4) in einem Abstand von 4 bis 8 cm von der Stimulationselektrode (3) in der Zuleitung (2) angeordnet ist.

14. Herzschrittmacher nach einem der vorangehenden Ansprüche, dadurch gekennzeichnet, dass der Temperaturfühler (4) mit einer Kalibrierschaltung zur Kalibrierung auf Absoluttemperatur verbunden ist.

**Claims**

1. A cardiac pacemaker having a control means for automatically adapting the pacing repetition rate to the patient's exercise, comprising a temperature sensor for detecting the temperature of the venous blood in the heart, which is disposed on a transvenous lead for a pacing electrode in the vicinity of the pacing electrode in such a way as to be capable of being introduced with the pacing electrode into the atrium or ventricle of the heart, and a logic circuit connected to the temperature sensor for controlling the pacing repetition rate in accordance with a stored characteristic as a function of the blood temperature detected by the temperature sensor, characterized in that the logic circuit (11) continuously determines the current change in time of the blood temperature from the blood temperatures detected by the temperature sensor (4), and in that the logic circuit (11) controls the pacing repetition rate in accordance with a heart rate-blood temperature basic characteristic (K2) corresponding to the state of physical rest when the change in time of the blood temperature does not exceed a certain minimum value, but in accordance with heart rate-blood temperature exercise characteristics (K1) corresponding to states of physical exercise when the change in time of the blood temperature exceeds this minimum value, whereby each of the exercise characteristics (K1) begins at a different working point on the basic characteristic (K2) and otherwise has a higher pacing repetition rate at one and the same blood temperature than the basic characteristic (K2), and all exercise characteristics (K1) consistently have a greater slope than the basic characteristic (K2).

2. The cardiac pacemaker as in claim 1, characterized in that the basic characteristic (K2) and the exercise characteristics (K1) are straight lines, the slope of the exercise characteristics (K1) being considerably greater than that of the basic characteristic (K2).

3. The cardiac pacemaker as in claim 2, characterized in that the slope of the exercise characteristics (K1) is within the range of 40 and 120 stimulations per minute and degree, and the slope of the basic characteristic (K2) is within the range of 5 and 25 stimulations per minute and degree.

4. The cardiac pacemaker as in claim 3, characterized in that the slope of the exercise characteristics (K1) is 80 stimulations per minute and degree, and the slope of the basic characteristic (K2) is 15 stimulations per minute and degree.

5. The cardiac pacemaker as in any of the above claims, characterized in that the slope of the exercise characteristics (K1) and the basic characteristic (K2) decreases in the range of the higher blood temperatures.

6. The cardiac pacemaker as in any of the above claims, characterized in that all exercise characteristics (K1) are parallel to each other.

7. The cardiac pacemaker as in any of the above claims, characterized in that the logic circuit (11) changes from the basic characteristic (K2) to the exercise characteristic (K1) starting at the particular working point on the basic characteristic (K2) or remains on the exercise characteristic (K1) when the change in time of the blood temperature exceeds an upper limiting value, and in that the logic control (11) changes from the exercise characteristic (K1) to the basic characteristic (K2) when the change in time of the blood temperature falls below a lower limiting value for a predetermined time span.

8. The cardiac pacemaker as in claim 7, characterized in that the upper limiting value is at 0.04 degrees per minute.

9. The cardiac pacemaker as in any of the above claims, characterized in that the cardiac pacemaker (1) contains an evaluation circuit (9) which determines in predetermined time periods the change in time of the blood temperature, the determined values as well as the blood temperature values being stored in a memory (10).

10. The cardiac pacemaker as in any of the above claims, characterized in that the transition between the characteristics (K1, K2) takes place without jumps in the pacing repetition rate.

11. The cardiac pacemaker as in any of the above claims, characterized in that the blood temperature detected by the blood temperature sensor (4) is periodically sensed at a predetermined repetition rate.

12. The cardiac pacemaker as in claim 11, characterized in that the repetition rate at which the detected blood temperature is sensed is changed in sympathy with the increase in time of the blood temperature.

13. The cardiac pacemaker as in any of the above claims, characterized in that the temperature sensor (4) is disposed at a distance of 4 to 8 cm from the pacing electrode (3) in the lead (2).

14. The cardiac pacemaker as in any of the above claims, characterized in that the temperature sensor (4) is connected with a calibration circuit for absolute temperature calibration.

—

## Revendications

1. Pacemaker comportant un dispositif de commande pour adapter la fréquence de répétition de stimulation aux efforts du patient, un palpeur de la température sanguine veineuse dans le cœur, ledit palpeur étant agencé sur une conduite d'amenée à une électrode de stimulation à proximité de ladite électrode de stimulation, de telle sorte que ledit palpeur peut être introduit avec l'électrode de stimulation dans l'oreillette ou le ventricule du cœur, et un circuit logique branché sur le palpeur de température, ledit circuit logique commandant la fréquence de répétition de stimulation suivant une ligne caractéristique mémorisée en fonction de la température sanguine mesurée par le palpeur de température, caractérisé en ce que le circuit logique (11) indique d'une façon continue la variation temporaire actuelle de la température sanguine sur base des températures sanguines mesurées par le palpeur de température (4) et que le circuit logique (11) commande la fréquence de répétition de stimulation dans le cas où la variation temporaire de la température sanguine ne dépasse pas une valeur minimale déterminée, selon une ligne de base (K2) de fréquence cardiaque – température sanguine, lorsque le corps est au repos, dans le cas où la variation temporaire de la température sanguine ne dépasse pas cette valeur minimale, selon au contraire des lignes de charge (K1) de fréquence cardiaque – température sanguine, lorsque le corps est soumis à des épreuves physiques, chacune des lignes de charge (K1) commençant à un point de travail différent sur la ligne de base (K2) et présentant autrement pour la même température respectivement une fréquence de répétition de stimulation plus élevée que la ligne de base (K2) et toutes les lignes de charge (K1) présentant sans interruption une pente plus grande que la ligne de base (K2).

2. Pacemaker selon la revendication 1, caractérisé en ce que la ligne de base (K2) et les lignes de charge (K1) sont des droites, la pente des lignes de charge (K1) étant beaucoup plus élevée que celle des lignes de base (K2).

3. Pacemaker selon la revendication 2, caractérisé en ce que la pente des lignes de charge (K1) se situe entre 40 et 120 stimulations par minute et degré et la pente de la ligne de base (K2) se situe entre 5 et 25 stimulations par minute et degré.

4. Pacemaker selon la revendication 3, caractérisé en ce que la pente des lignes de charge (K1) comporte 80 stimulations par minute et degré et la pente de la ligne de base (K2) comporte 15 stimulations par minute et degré.

5. Pacemaker selon l'une des revendications précédentes, caractérisé en ce que la pente des lignes de charge (K1) et de la ligne de base (K2) décroît dans la zone des températures sanguines plus élevées.

6. Pacemaker selon l'une des revendications précédentes, caractérisé en ce que toutes les lignes de charge (K1) sont parallèles les unes aux autres.

7. Pacemaker selon l'une des revendications précédentes, caractérisé en ce que le circuit logique (11) passe de la ligne de base (K2) à la ligne de charge (K1), qui part du point de travail correspondant sur la ligne de base (K2) ou demeure sur la ligne de charge (K1), lorsque la variation temporaire de la température sanguine dépasse une limite supérieure, et que le circuit logique (11) passe de la ligne de charge (K1) à la ligne de base (K2), lorsque la variation temporaire reste inférieure à la limite inférieure pour un laps de temps déterminé.

8. Pacemaker selon la revendication 7, caractérisé en ce que la limite supérieure est de l'ordre de 0,04 degré par minute.

9. Pacemaker selon l'une des revendications précédentes, caractérisé en ce que le pacemaker (1) contient un circuit d'évaluation (9) qui détermine la variation temporaire de la température sanguine dans des laps de temps déterminés, les valeurs déterminées étant mémorisées dans une mémoire (10) tout comme les valeurs de la température sanguine.

10. Pacemaker selon l'une des revendications précédentes, caractérisé en ce que le passage entre les lignes (K1, K2) se fait sans discontinuité de la fréquence de répétition de stimulation.

11. Pacemaker selon l'une des revendications précédentes, caractérisé en ce que la température sanguine mesurée par les palpeurs de température sanguine (4) est lue périodiquement à une fréquence de répétition déterminée.

12. Pacemaker selon la revendication 11, caractérisé en ce que la fréquence de répétition avec laquelle la température sanguine est mesurée, est modifiée dans le même sens que la montée temporaire de la température sanguine.

13. Pacemaker selon l'une des revendications précédentes, caractérisé en ce que le palpeur de température (4) est disposé dans la conduite (2) à une distance de 4 à 8 cm de l'électrode de stimulation (3).

14. Pacemaker selon l'une des revendications précédentes, caractérisé en ce que le palpeur de température (4) est relié à un circuit de calibrage pour calibrer à la température absolue.

*Fig. 1*

*Fig. 2*

*Fig.3*

Fig. 4